# EUROPEAN PATENT SPECIFICATION

(11) **EP 2 305 862 B1**
(45) Date of publication and mention of the grant of the patent: **06.01.2016**
(21) Application number: 09742444.4
(22) Date of filing: 08.05.2009
(51) Int. Cl.: A61L 15/62, D01F 8/06, A61F 13/15, A61L 15/60, A61L 15/26

(54) **ENVIRONMENTALLY-FRIENDLY DISPOSABLE ABSORBENT ARTICLE**
UMWELTFREUNDLICHER SAUGFÄHIGER EINWEGARTIKEL
ARTICLE ABSORBANT JETABLE, RESPECTUEUX DE L'ENVIRONNEMENT

(30) Priority: 09.05.2008 MX 2008006155
(43) Date of publication of application: 06.04.2011
(73) Proprietor: Grupo P.I. Mabe, S.A. de C.V., 72230 Puebla (MX)
(72) Inventor: CANALES ESPINOSA DE LOS MONTEROS, Carlos, 72230 Puebla (MX); ZAMUDIO AHUMADA, Andrés, 72230 Puebla (MX); SÁNCHEZ FERNÁNDEZ, Lucia, 72230 Puebla (MX)
(74) Representative: Gil-Vega, Victor
(86) International application number: PCT/IB2009/005526
(87) International publication number: WO 2009/136271

(56) References cited:
- EP-A1- 1 518 526
- WO-A1-00/01335
- WO-A1-00/19949
- WO-A1-02/083192
- WO-A1-2007/106929
- WO-A1-2009/012284
- WO-A1-2009/012284
- WO-A2-01/39807
- WO-A2-01/39807
- US-A- 4 798 603
- US-A- 5 185 009
- US-B1- 6 509 092
- US-B1- 6 509 092

## Description

### Background of the Invention

A disposable absorbent article is a product that is used to absorb and contain body exudates and which is discarded after use, like disposable baby diapers, disposable underwear such as training pants and swimming pants , and all kinds of adult incontinence, sanitary napkins and panty liners, among others.

The disposable absorbent articles are used to protect the wearer's clothing, preventing it from getting dirty with body waste. The use of such articles has become indispensable in human daily life. However, most of the materials used to manufacture these disposable items are not sustainable neither biodegradable, it takes hundreds of years return them to nature.

A sustainable material is based on renewable resources, such that it helps to satisfy the needs of present generations without committing the resources of future generations, while a biodegradable material is that which is made from naturally occurring compounds, so that after certain period of time it breaks down and transforms in less complex compounds, until it reaches its most basic components.

The disposable absorbent articles such as disposable diapers, are generally made of a permeable top sheet, an impermeable back sheet (which may consist of two or more films laminated together), an absorbent core placed between the top and back sheets containing fibres of absorbent material and particles of superabsorbent material, external barrier cuffs, a fastening system, means of elastification in waist and legs areas, and union means, the article may also contain one or more transfer layers, one or more wrap layers and one or more elastic zones, in addition to perfume and / or some kind of formula for skin care and for control bad odour. Most of these components are made from polymers derived from petroleum, such as polyethylene, polypropylene, polyester, etc.. For purposes of the present invention, a component of the article that contains such polymers will be referred as polymeric components of the article. Thus, the polymeric components of a disposable absorbent article, such as a disposable diaper are: top sheet, back sheet, fastening system, barrier cuffs, transfer layers, wrap layers, elastic leg and waist bands; that is to say, approximately 80% of the components of a disposable absorbent article, such as a disposable diaper, are polymeric components, and are neither sustainable nor biodegradable.

In addition, the superabsorbent material commonly used in the absorbent core of such absorbent articles, is generally a sodium or potassium polyacrylate, material not sustainable and not biodegradable.

All the materials can be joined using ultrasonic bonds, by means of heat and pressure, or using hot melt adhesives. In the latter case, the adhesives are neither sustainable nor biodegradable.

Thus, a disposable absorbent article, such as a disposable diaper, contains between 8 and 15 components, in addition to the bag in which the articles are packed. Based on eight components, namely, top sheet, back sheet, barrier cuffs, absorbent core, superabsorbent material, fastening system and union means, currently only one of them, the absorbent material, is sustainable and biodegradable, i.e. 1/8 or 12.5%.

The present invention relates to a disposable absorbent article in which at least 50% of the components are sustainable or biodegradable materials that is to say, a disposable absorbent article environmentally friendly, in addition this article satisfies consumer expectations in terms of absorbency, comfort and cost. An important aspect of the present invention is that the materials used are suitable for processing them at high production rates used on a daily basis for the manufacture of such articles.

There are many developments and proposals for use of biodegradable materials in absorbent articles. Some of the most important are described in patents and publications U.S. 4,964,857, US5, 185.009, WO 07/106929, WO 07/106929, US5, 437.918, US2005/0112363, US6, 506.873, JP 07008520, U.S. 5417679, WO 01 / 39 807. U.S. 5,295,985. U.S. 5,939,467, WO 02/028444, WO 05/094910, WO 99/33420 and the application MX 9,911,256

However the large number of developments in this field, it is difficult to find in the market environmentally friendly disposable absorbent articles. This is because the available materials:
- Are not suitable for use in the manufacturing of such items (do not have the needed strength or the needed weight)
- Do not have the right characteristics in terms of softness, flexibility and security for the user.
- Are expensive compared with commonly used materials.

For example, U.S. Patent 4,964,857 of Charles Osborne, entitled "Biodegradable Disposable Diaper" ; refers to a diaper that has a renewable top sheet and a back sheet of paper coated with wax, which helps prevent fluid to flow through itself. However, it is clear that a product of this nature is not sure and not comfortable for the user, disposable absorbent articles with these characteristics have no place in the market. Another development in this regard is U.S. Patent 5,185,009 of Elmo Sitman, which describes a biodegradable diaper with a top sheet, a back sheet, an absorbent core and adhesives; adhesive tapes are made of cellulose and covered with a biodegradable adhesive; the top sheet can be biodegradable rayon or polypropylene, the back sheet can be a biodegradable polyethylene, the bonding adhesive is latex rubber, the core layer is absorbent wood pulp or a copolymer of poly-acrylic-nitrite and retaining straps are coated paper tape. A diaper of this type not has been marketed as such, since the described materials are not adequate to provide the consumer an article with the features he expects in terms of absorbency, comfort and safety. The international publication WO 06/065110, of Estela Concepción González Flores, refers to a disposable absorbent article which uses a natural waterproofing agent applied to a film, such as paper or nonwoven fabric, the article also uses a re-useable waterproof covering. The international publication WO 07/106929, of Tristano PTY LTD, describes a disposable absorbent article fabricated with biodegradable materials made from one or more biodegradable polymers such as poly-Capra-lactone and mixtures of starch and PLA, the article contains others seeds embedded in the absorbent core, so that after used, it is buried and the seeds germinate. All remaining components of the article, such as elastic and adhesive fastening system may also be biodegradable.

The present invention provides a disposable absorbent article, such as a disposable diaper, a training pants, a pad or any similar article in which at least half of its components contain sustainable or biodegradable materials; the article is comfortable, safe and affordable, plus it can be manufactured on production lines of high speed.

So in the absorbent article of the present invention, all polymeric components are replaced by polymeric materials that can be chosen from:
- Films or materials containing polylactic acid (PLA) or polyhydroxy-alcanoate (PHA)
- Films or materials containing natural fibres such as cotton or rayon.
- Films or polymeric materials containing a pro-degrading agent.

Moreover, the superabsorbent material, which is commonly sodium or potassium polyacrylate, is replaced by a mixture containing at least 20% of absorbent polymer from corn starch particles in combination with polyacrylate. Thus, is available to the consumer, a environmentally friendly product that has the right features in absorption, comfort, safety and smoothness, at an affordable cost and also it can be manufactured on production lines at high speed.

In what refers to the use of natural fibres such as cotton or rayon to produce films that can be used in this articles, it is noteworthy that there are some developments in nonwoven fabric containing cotton fibres, however most refers to fabrics suitable for cleaning, due to their great absorbent capacity, such as described in WO 91/08333 which protect a method for manufacturing hydrophilic nonwoven fabric containing natural fibres, specifically unbleached cotton, a fabric of this type is not suitable for be used as top layer of a disposable absorbent article due to its high degree of hydrophilicity. In disposable absorbent articles it is desirable the use of a permeable top layer to allow the passage of fluid but it should not remain wet, meaning that the liquid passes through and is distributed, absorbed and retained by inner layers of the article, keeping moisture away from the user's skin; a nonwoven fabric as described in WO 91/08333, absorbs and retains moisture, so it is not appropriate for its use in absorbent articles.

Other developments of nonwoven fabric containing natural fibres are used as absorbent material, either in disposable sanitary articles, or in other products, such as food packaging, etc.., Fabrics suitable for this use are protected in U.S. Patent 5,958,186 SCA Hygiene Products and U.S. Patent Application 2003/007026 Jens O. Brøchner.

On the other hand, non-woven fabrics containing natural fibres can be found as filling for mattresses, duvets, pillows, etc.. this type of fabrics are described in U.S. Patent Application 2003/0021978 by Anthony Wolf.

None of the films described is suitable for use as a top sheet of disposable sanitary articles. We propose the use in a disposable absorbent article of a nonwoven fabric containing at least 15% of a natural fibres such as cotton, mixed with other synthetic fibres, so that the fabric is permeable but does not retain moisture, has adequate resistance to withstand the stresses to which it is subjected during the manufacturing process and during use, plus it is smooth.

### Summary of the invention

The main object of the present invention is to provide the consumer with an environmentally friendly disposable absorbent article, in which at least half of the materials used to manufacture them, contain natural or biodegradable compounds, therefore, raises several additional objectives:
- The use as top sheet of the article, a nonwoven fabric containing cotton.
- The use as top sheet of the article, a nonwoven fabric containing polylactic acid (PLA) or poly-hydroxy-alcanoates (PHA).
- The use as top sheet of the article, a nonwoven fabric containing a pro-degrading agent.
- The use as back sheet of the article, a waterproof film containing polylactic acid (PLA) or poly-hydroxy-alcanoates (PHA) .
- The use as back sheet of the article, a polyethylene containing a pro-degrading
- The use as back sheet of the article, a laminated layer of a film containing PLA, PHA or a pro-degrading agent and :
   A nonwoven fabric containing cotton.
   A nonwoven fabric containing PLA or PHA
   A nonwoven fabric containing a pro-degrading agent.
- The use of superabsorbent material containing at least 20% of natural and / or biodegradable particles.
- The use of a fastening system containing PLA or PHA.
- The use of a fastening system containing a pro-degrading agent.
- The use of films containing PLA or PHA or a pro-degrading agent for barrier cuffs, transfer layers and wrap layers.
- The use of Adhesives containing PHA or PLA.
- The use of elastic or elastomeric materials containing PLA, PHA or pro-degrading agent.

### Description of the Drawing

The invention will be described with reference to the accompanying drawing, which illustrates a presently preferred embodiment of the invention.

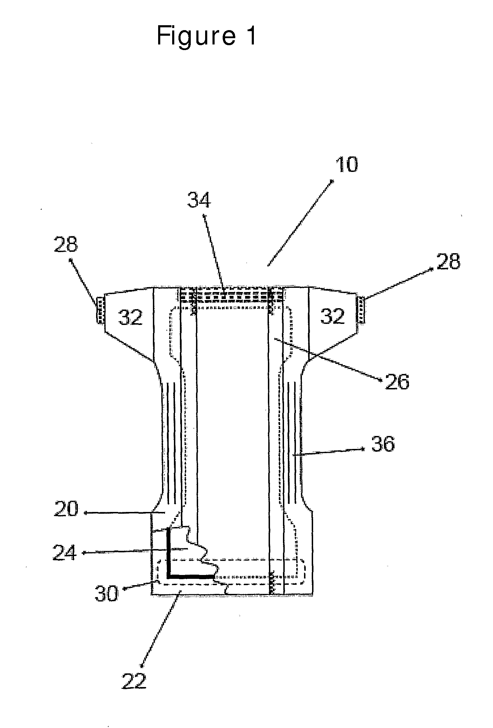

Figure 1 shows a disposable absorbent article.

### Detailed Description of the Invention

The term "disposable absorbent article" refers to articles which absorb and retain body exudates and are discarded after use. It is protected by the present invention a disposable absorbent article which is friendly to nature, such that at least 50% of the total materials used to manufacture them, contain natural and / or biodegradable compounds.

This description will be based on a disposable baby diaper, understanding that can be applied to other disposable absorbent articles such as disposable diapers for incontinent adults, children's training pants, panty-liners, sanitary napkins or any other disposable absorbent article.

As seen in Figure 1, the top sheet (20) of article (10) of the invention is preferably a nonwoven fabric containing at least about 50% of PLA or PHA, and the rest of a polyolefin, such as polyethylene or polypropylene; as mentioned in the background, the nonwoven containing PLA or PHA is currently available in the market. However, this kind of film is not suitable for use as top sheet of a disposable absorbent article, mainly because the PLA and PHA do not provide the necessary smoothness for this type of articles, besides, to be affordable, the weights of the films used as top sheet are handled in 10 to 15 g/m². Available nonwoven fabrics having this weight and made of degradable polymers such as PLA or PHA, do not have the strength and elongation required to work well in the processes of producing such articles. In addition, disposable absorbent articles, during use, are in direct contact with the skin all day and night, the films made from polymers such as PLA and PHA do not have the smoothness required for no hurt the skin. Having in mind the aforesaid, the nonwoven fabric used as top sheet (20) in article (10) of this invention is made from bicomponent fibres of sheath-core type, the core contains about 50 to about 100% of PLA or PHA, preferably from about 80 to about 100% of PLA or PHA while the sheath contains about 50 to about 100% polypropylene, preferably from about 80 to about 100% polypropylene, the remaining contents of the sheath may be PLA or PHA. This nonwoven fabric used as top sheet (20) in Article (10) invention contains at least 50% of sustainable materials.

For the purposes of the present invention, it can also be used as top sheet, a nonwoven fabric containing natural fibres, such as cotton. In this regard, there are several developments, however, no such fabrics are used in disposable absorbent articles, since cotton is a hydrophilic fibre, so it absorbs and retains liquid, undesirable feature in the top sheet of a disposable absorbent article, therefore nonwoven fabrics containing cotton are used as cleaning cloths, for food packaging, as absorbent pads or even as a filling for mattresses. On the other hand, for the manufacture of absorbent disposable articles is required that the film used as top sheet and / or back sheet, has certain characteristics of machine direction and cross direction strength; a nonwoven fabric containing cotton, has less strength that a nonwoven fabric without it, a greater amount of cotton fibres in a non woven fabric, less strength. Thus, for the purposes of this invention, can be used as top sheet a nonwoven fabric containing between about 15 and 50% cotton fibre which are natural and biodegradable fibres blended with polyester or polypropylene.

It can also be used as top sheet of the article of this invention, a nonwoven fabric made from polyolefins such as polypropylene or polyester which has added a pro-degrading agent, preferably the pro-degrading agent contains saponified fatty acids and calcium, so the film can be degraded in the presence or absence of oxygen, i.e. to compost or garbage dumps.

The back sheet (22) of article (10) of the present invention should be soft, flexible and waterproof, generally, is used low density polyethylene. For the purposes of article (10) of the invention, can be used as back sheet a low density polyethylene film containing a pro-degrading agent, preferably containing saponified fatty acids and calcium, so the film can be degraded in the presence or the absence of oxygen. It can also be used as backsheet or the article (10), a waterproof film made from PLA and / or PHA.

It is desirable that the back sheet (22) of article (10) has the appearance and softness of a fabric, to achieve this, is used a laminate of two films: polyethylene as described in the preceding paragraph, and nonwoven fabric as described for the top sheet (20).

The absorbent core (24) of article (10) of the invention, is constituted of fibres of absorbent material and particles of superabsorbent material; the absorbent material fibres are cellulosic fibres and the superabsorbent material particles are composed of a mixture of particles of sodium or potassium polyacrylate and at least 20% of absorbing particles derived from corn starch, this natural absorbent particles help the rapid dispersion of fluid in the core making it more efficient. Thus, in the absorbent core (24) of article (10) of the present invention, at least 20% of the particles of superabsorbent material are natural and biodegradable.

Another element of the disposable absorbent article (10) of the invention are the external barriers cuffs (26), these are made up of some of the films described above for the top sheet (20) with the proper treatment of hydrophobicity required for this element of the article.

The fastening system of disposable diapers is usually formed by a pair of fastening tapes (28), and a front band or tape (30); fastening tapes (28) are placed on the back of the diaper waist zone, while the front band or tape is placed on the outer layer on the frontal waist zone of the article. It can be used a mechanical (Hook & Loop) or adhesive system, in either case, both down straps or tapes as the front band, are usually made of no sustainable or biodegradable materials, usually polyethylene or polypropylene, so, for the fastening system of the present invention are used preferably a couple of bands (28) (mechanical or adhesive) and a front band or tape (30), which are made from degradable polymers such as PHA and PLA, or from polyethylene or polypropylene containing a pro-degrading agent; preferably the pro-degrading agent contains saponified fatty acids and calcium.

An absorbent article may contain one or more transfer layers and one or more wrap layers, for the absorbent article of the present invention, as transfer or wrap layers are used nonwoven films such as those described for the upper layer.

Moreover, the absorbent article has elastic means (36 and 34) at least in the part that is in contact with the wearer's legs and in the part of the waist, for this purpose, usually, are used lycra, polyester elastic bands, films of polypropylene or polyurethane, which are bonded, in a state of tension, between two layers of the article, generally between the top and backsheet layers; for the purposes of the present invention, as elastic means in legs or waist zones, can be used latex or natural rubber; can also be used elastic materials made from polyolefin plus a pro degrading agent.

Article (10) may also have other stretch zones located in different parts of the article, for example in the sides zones, in the ears (32) or as an integral part of the fastening bands (28). For the article (10) of the invention, these elastic areas could be formed by a laminate consisting of a couple of films containing, between them, an elastomeric material, which can be bonded to the films with adhesive, ultrasonic, thermal bond or extruded directly onto one of the films. For the purposes of the invention, the elastomeric material contains a pro-degrading agent and the films are films like those described for topsheet or backsheet of the article (10) of the invention.

To bond all the components of the article (10), can be used thermal, ultrasonic or adhesive bonds. The components of the absorbent disposable articles commonly found on the market, are bonded by means of adhesives, specifically hot melt and fast dry adhesives. For the purposes of the invention can be used adhesives made from degradable polymers such as PLA or PHA.

As additional elements, absorbent articles often contain perfume and / or a formula to control odour and / or a formula to protect the user's skin; for the article of the invention, is used an essential oil blend containing chamomile, lavender, geranium, ylang ylang and eucalyptus to protect user's skin and cupric chlorophyll as an agent for odour control, all naturally occurring compounds.

### Examples

### Example 1:

**Top Sheet:** nonwoven fabric of bi-component fibres (PLA core covered with polypropylene), with a weight of 13 g/m2.

**Back Sheet:** laminated film formed of 15 g/m2 biodegradable polyethylene (polyethylene + pro degrading agent containing saponified fatty acids and calcium) and nonwoven fabric of 13 g/m2 composed of polypropylene /cotton fabric in a 70/30 ratio.

**Absorbent Material:** cellulose fibres.

**Superabsorbent Material:** a mixture of polyacrylate particles and particles of corn starch in a ratio 80 / 20.

**External Barrier cuffs:** nonwoven fabric of 13 g/m2 composed of bicomponent fibres (PLA core covered with polypropylene).

**Emollient:** Formula based on vegetable oils, mainly avocado oil and sweet almond oil.

**Fastening System :** mechanical Hook & Loop fastening system, made of polyolefins.

**Leg and Waist elastic :** lycra

**Union means:** hot melt adhesives.

Whereas the back sheet is formed by two films joined together and that the fastening system is formed by fastening bands and frontal band , the diaper is formed by a total of 11 elements, seven of which contain sustainable or biodegradable compounds, i.e. 63.6% of the components of the disposable diaper of Example 1 contain sustainable or biodegradable compounds.

### Example 2:

**Top Sheet:** non-woven fabric of 15 g/m2 composed of 20% Cotton fibres and 80% polypropylene fibres.

**Back Sheet ;** polyethylene of 15 g/m2 plus a pro-degrading agent containing saponified fatty acids and calcium, laminated with a non-woven fabric of 13 g/m2 composed of polypropylene / cotton fibres in a 70/30 ratio. **Absorbent Material:** cellulose fibres.

**Superabsorbent Material:** a mixture of polyacrylate particles and particles of corn starch in a 80 / 20 ratio.

**External Barrier Cuffs:** nonwoven fabric of 13 g/m2 composed of bicomponent fibres (PLA core covered with polypropylene).

**Emollient:** Formula based on chamomile oil, lavender oil, geranium oil, ylang ylang oil and eucalyptus oil plus cupric chlorophyll to control odours. **Fastening System :** Hook & Loop mechanical system made of polyolefins plus a pro-degrading agent.

**Legs and waist elastics**: lycra.

**Union means:** Hot melt adhesives made from PLA.

Total components: 11 . Sustainable or biodegradable materials: 9 = 81.8%

### Example 3:

**Top Sheet:** non-woven fabric of 13 g/m2 composed of 20% Cotton and 80% polypropylene fibres.

**Back Sheet :** 13 g/m2 polyethylene containing a pro-degrading agent, laminated with a non-woven fabric of 13 g/m2 with 20% of cotton fibres. **Absorbent Material:** cellulose fibres.

**Superabsorbent Material:** a mixture of polyacrylate particles and particles of corn starch in a ratio 80 / 20.

**Transfer Layer:** polypropylene nonwoven fabric plus a pro-degrading agent.

**External Barrier Cuffs:** nonwoven fabric of 13 g/m2 composed of bicomponent fibres (PLA core covered with polypropylene).

**Emollient:** Formula formed with essential oils.

**Fastening System :** Hook & Loop mechanical fastening system made of polyolefins plus a pro-degrading agent.

**Union means**: Hot melt adhesives

Total component: 12. Sustainable or biodegradable materials: 11 = 91.6%

### Example 4:

**Top Sheet:** nonwoven fabric of 13 g/m2 composed of 20% Cotton and 80% polypropylene fibres.

**Back Sheet:** 13 g/m2 laminated film composed of polyethylene containing a pro-degrading agent and non-woven fabric of 13 g/m2 with 20% of cotton fibres.

**Absorbent Material:** cellulose fibres.

**Superabsorbent Material:** a mixture of polyacrylate particles and particles of corn starch in a ratio 80 / 20.

**Wrap Layer:** paper of 15 g/m2.

**Transfer Layer:** Polypropylene non-woven fabric of 18 g/m2 containing a pro-degrading agent.

**External Barrier Cuffs:** nonwoven fabric of 13 g/m2 composed of bicomponent fibres (PLA core covered with polypropylene).

**Emollient:** Formula containing essential oils and chlorophyll.

**Fastening System :** Hook & Loop mechanical fastening system made of polyolefins containing a pro-degrading agent.

**Stretch Zones:** biodegradable elastomer (polyolefin plus a por-degradent agent) laminated between two layers of nonwoven fabric of bi-component fibres (PLA core covered with polypropylene) with a weight of 13 g/m2. **Legs and waist elastic**: lycra or polyurethane foam plus a pro-degrading agent.

**Union means:** Hot melt adhesives based on PLA and / or PHA polymers.

Total components: 14 Sustainable or biodegradable materials: 14 = 100%

## Claims

1. A disposable absorbent article, comprising a permeable top sheet, an impermeable back sheet, an absorbent core placed between the top and back sheets containing absorbent material fibres and particles of superabsorbent material, external barriers cuffs, waist and leg elastic means, a fastening system and union means; **characterised in that** at least 50% these items contain biodegradable compounds, being such biodegradable compounds made from naturally occurring compounds, so that after certain period of time they break down and transform in less complex compounds, until they reach their most basic components, and wherein the biodegradable compounds are selected from:
for the top sheet of the article: a nonwoven fabric containing cotton, a nonwoven fabric containing polylactic acid (PLA) or poly-hydroxy-alcanoates (PHA) or a nonwoven fabric containing a pro-degrading agent;
for the back sheet of the article: a waterproof film containing polylactic acid (PLA) or poly-hydroxyalcanoates (PHA) or a polyethylene containing a pro-degrading agent; or a laminated layer of a waterproof film and a nonwoven fabric wherein the waterproof film includes PLA, PHA or a polyethylene containing a pro-degrading agent and the nonwoven fabric includes PLA, PHA, cotton or a pro-degrading agent;
as absorbent core material cellulose fibers as superabsorbent material: particles containing at least 20% of biodegradable particles;
as barrier cuffs: a nonwoven fabric containing PLA, PHA or a pro-degrading agent;
as elastic means: latex, natural rubber or elastic materials containing polyolefin plus a pro-degrading agent;
as fastening system: a couple of bands and a front band made from PHA or PLA or polyethylene or polypropylene containing a pro-degrading agent;
as union means: adhesives containing PHA or PLA.
and wherein the top sheet of the article is a nonwoven fabric with a weight between 10 and 15 gr/m2 formed from bicomponent fibers of the core and sheath type, with the core containing 80-100% of polylactic acid and the sheath containing 80-100% of polypropylene.

2. A disposable absorbent article as described in prior claim, **characterised in that** the impermeable back sheet is a film of low density polyethylene with a weight of about 10-25 g/m2, containing a pro-degrading agent which contains saponified fatty acids and calcium.

3. A disposable absorbent article as described in any of claims 1-2, **characterised in that** the impermeable back sheet is a film made from polylactic acid.

4. A disposable absorbent article as described in any of claims 1-2, **characterised in that** the impermeable back sheet is a film formed from polhydroxyalcanoates.

5. A disposable absorbent article as described in claims 1-2, **characterised in that** the impermeable back sheet is laminated with a nonwoven fabric of about 10 and 15 g/m2 and containing bicomponent core and sheath fibres, with the core containing about 80 to about 100% polylactic acid and the sheath containing about 80 to 100% polypropylene.

6. A disposable absorbent article as described in claims 1-2, **characterised in that** the impermeable back sheet is laminated to a nonwoven fabric of about 10 and 15 g/m2 and containing bicomponent core and sheath fibres, with the core containing about 80 to about 100% of poly-hydroxy-alcanoate and the sheath containing about 80 to 100% polypropylene.

7. A disposable absorbent article as described in claims 1-2, **characterised in that** the impermeable back sheet is laminated to a nonwoven fabric of about 10 and 15 g/m2 containing at least about 20% of cotton fibres.

8. A disposable absorbent article as described in any of the preceding claims, **characterised in that** the superabsorbent core is formed of cellulose fibres and two kinds of particles, homogeneously mixed:
a) Particles of sodium or potassium polyacrylate
b) Absorbent particles derived from corn starch

9. A disposable absorbent article as described in claim 8, **characterised in that** the mixture contains about 50 to about 95% of particles of sodium or potassium polyacrylate and about 5 to about 50% of absorbent particles derived from corn starch.

10. A disposable absorbent article as described in any of the preceding claims, **characterised in that** the external barriers cuffs are formed from a film of nonwoven fabric with a weight between about 10 and about 15 g/m2, formed of bicomponent core and sheath fibres, with the core containing about 80 to about 100% polylactic acid and the sheath containing about 80 to about 100% polypropylene.

11. A disposable absorbent article as described in any of claims 1 to 10, **characterised in that** the external barriers cuffs are formed from a nonwoven fabric with a weight of about 10 to about 15 g/m2, formed of bicomponent core and sheath fibres, with the core containing about 80 to about 100% poly-hydroxy-alcanoates and the sheath containing about 80 to about 100% polypropylene.

12. A disposable absorbent article as described in any of claims 1 to 10, **characterised in that** the external barriers cuffs are formed from a nonwoven film with a weight of about 10 to about 15 g/m2 containing at least 15% of cotton fibres.

13. A disposable absorbent article as described in any of the preceding claims, **characterised in that** the leg and waist elastic means of the article is latex or natural rubber.

14. A disposable absorbent article as described in any of claims 1 to 12, **characterised in that** the legs and waist elastic means of the article is made from polyolefins plus a pro-degrading agent.

15. A disposable absorbent article as described in any of the preceding claims, **characterised in that** the fastening system of the article consists of a pair of fastening tapes placed on the rear waist and a band placed on the outside of the front waist portion thereof, such that fastening tapes and / or the band are formed of polyolefins plus a pro-degrading agent containing saponified fatty acids and calcium.

16. A disposable absorbent article as described in any of claims 1 to 14, **characterised in that** the fastening system consists of a pair of fastening tapes placed on the rear waist of the item and a band placed on the outside of the front waist portion thereof, such that fastening tapes and / or the band containing polylactic acid or poly-hydroxy-alcanoates.

17. A disposable absorbent article as described in any of the preceding claims, **characterised in that** it contains stretch zones which consist of an elastomeric material placed between two films; the elastomeric material is a polyolefin plus a pro-degrading agent containing saponified fatty acids and calcium and the films are nonwoven films.

18. A disposable absorbent article as described in any of claims 1 to 16, **characterised in that** it contains stretch zones which consist of an elastomeric material placed between two films; the elastomeric material is a polyolefin plus a pro-degrading agent containing saponified fatty acids and calcium and the films are nonwoven fabrics of about 10-15 g/m2, which are formed by bicomponent core and sheath fibres, with the core containing about 80 to about 100% of polylactic acid and the sheath containing of about 80 to 100% of polypropylene.

19. A disposable absorbent article as described in any of claims 1 to 16, **characterised in that** it contains stretch zones which consist of an elastomeric material placed between two films; the elastomeric material is a polyolefin plus a pro-degrading agent containing saponified fatty acids and calcium and the films are nonwoven fabrics of about 10-15 g/m2, which are formed by bicomponent core and sheath fibres, with the core containing about 80 to about 100% of poly-hydroxy-alcanoate and the sheath containing of about 80 to 100% of polypropylene.

20. A disposable absorbent article as described in any of claims 1 to 17, **characterised in that** it contains stretch zones which consist of an elastomeric material placed between two films; the elastomeric material is a polyolefin plus a pro-degrading agent containing saponified fatty acids and calcium and the films are nonwoven fabrics of about 10-15 g/m2, containing at least about 20% of cotton fibres.

21. A disposable absorbent article as described in any of the preceding claims, **characterised in that** it contains a composition for skin care which is a formula containing vegetable oils, mainly avocado oil and sweet almond oil.

22. A disposable absorbent article as described in any of the preceding claims, **characterised in that** it contains a composition for skin care which is a formula containing a mixture of essential oils.

23. A disposable absorbent article as described in any of the preceding claims, **characterised in that** it contains chlorophyll or chlorophyll derivatives as a means of odour control.

## Patentansprüche

1. Ein absorbierender Einwegartikel mit einer durchlässigen Oberschicht, einer undurchlässigen Unterschicht, einem absorbierenden Kern zwischen der Ober- und Unterschicht, der Fasern aus absorbierendem Material enthält sowie Teilchen eines hochabsorbierenden Materials, mit äusseren Sperrschichtenn, elastischen Mitteln für Unterkörper und Beine, mit einem Befestigungssystem und Verbindungsmitteln, **dadurch gekennzeichnet, dass** mindestens 50% dieser Artikel biologisch abbaubare Verbindungen enthält, wobei diese biologisch abbaubaren Verbindungen aus in der Natur vorkommenden Verbindungen hergestellt werden, so dass sich dieselben nach einer gewissen Zeit auflösen und in weniger komplexe Verbindungen umwandeln bis sie wieder den Zustand ihrer Ausgangskomponenten erreichen, von denen die biologisch abbaubaren Verbindungen ausgewählt wurden:
Für die Oberschicht des Artikels: eine nichtgewebte Struktur mit Baumwolle, eine nichtgewebte Struktur mit Polymilchsäure (PLA) oder Poly-Hydroxy-Alcanoaten (PHA) oder eine nichtgewebte Struktur mit einem degradierungsfördernden Agens;
für die Unterschicht des Artikels: ein wasserdichter Film, der Polymilchsäure (PLA) oder Poly-hydroxyalkanoate (PHA) oder ein Polyethylen mit einem degradierungsfördernden Agens enthält. Oder eine laminierte Schicht eines wasserdichten Films und eine nichtgewebte Struktur, wobei die wasserdichte Schicht PLA, PHA oder ein Polyethylen mit einem degradierungsfördernden Agens umfasst und die nichtgewebte Struktur PLA, PHA, Baumwolle oder einen degradierungsfördernden Agens enthält;
ein absorbierendes Kernmaterial: Zellulosefasern;
als hochabsorbierendes Material: Teilchen mit einem Anteil von mindestens 20% biologisch abbaubaren Teilchen;
als Sperrschichtenn: eine nichtgewebte Struktur, die PLA, PHA oder einen degradierungsfördernden Agens enthält;
als elastisches Mittel: Latex, Naturkautschuk oder elastische Werkstoffe, die Polyolefin zusammen mit einem degradierungsfördernden Agens enthalten;
als Befestigungssystem: ein Paar Streifen und ein Vorderband aus PHA oder PLA oder
Polyethylen oder Polypropylen mit einem degradierungsfördernden Agens;
als Verbindungmittel: Klebstoff mit PHA oder PLA;
und dadurch dass die Oberschicht des Artikels eine nichtgewebte Struktur mit einem Gewicht von 10 bis 15 g/m² ist, gebildet aus Bikomponentenfasern des Kerns und in Form einer Ummantelung, wobei der Kern von 80 bis 100 % Polymilchsäure und die Folie zwischen 80 und 100% Polypropylen enthält.

2. Ein absorbierender Einwegartikel übereinstimmend mit der Beschreibung des vorhergehenden Anspruchs **dadurch gekennzeichnet, dass** die undurchlässige Unterschicht ein Film ist mit einem Polyethylen geringer Dichte mit einem Gewicht von ca. 10-25 g/m², der einen degradierungsfördernden Agens mit verseiften Fettsäuren und Kalzium enthält.

3. Ein absorbierender Einwegartikel übereinstimmend mit einem der beiden Ansprüche 1-2 **dadurch gekennzeichnet, dass** die undurchlässige Unterschicht ein Film aus Polymilchsäure ist.

4. Ein absorbierender Einwegartikel übereinstimmend mit einem der beiden Ansprüche 1-2 **dadurch gekennzeichnet, dass** die undurchlässige Unterschicht ein Film aus Polyhydroxyalkanoaten ist.

5. Ein absorbierender Einwegartikel übereinstimmend mit den Ansprüchen 1-2 **dadurch gekennzeichnet, dass** die undurchlässige Unterschicht mit einer nichtgewebten Struktur von ca. 10 und 15 g/m² laminiert ist und einen Bikomponentenkern und Ummantelungsfasern umfasst, wobei der Kern ca. 80 bis ca. 100% Polymilchsäure enthält und die Ummantelung ca. 80 bis 100% Polypropylen.

6. Ein absorbierender Einwegartikel übereinstimmend mit den Ansprüchen 1-2 **dadurch gekennzeichnet, dass** die undurchlässige Unterschicht zu einer nichtgewebten Struktur von ca. 10 und 15 g/m² laminiert wurde und einen Bikomponentenkern und Ummantelungsfasern enthält, wobei der Kern ca. 80 bis ca. 100% Polyhidroxyalkanoat enthält und die Ummantelung ca. 80 bis 100% Polypropylen.

7. Ein absorbierender Einwegartikel übereinstimmend mit den Ansprüchen 1-2 **dadurch gekennzeichnet, dass** die undurchlässige Unterschicht zu einer nichtgewebten Struktur von ca. 10 und 15 g/m² mit mindestens etwa 20% Baumfollfasern laminiert wird.

8. Ein absorbierender Einwegartikel übereinstimmend mit irgendeinem der vorhergehenden Ansprüche **dadurch gekennzeichnet, dass** der hochabsorbierende Kern aus Zellulosefasern und zwei Teilchenarten gebildet wird, die gleichmässig gemischt werden:
a) Natriumteilchen oder Teilchen aus Kaliumpolyakrylat
b) Absorbierende Teilchen auf der Basis von Weizenstärke.

9. Ein absorbierender Einwegartikel übereinstimmend mit Anspruch 8 **dadurch gekennzeichnet, dass** die Mischung ca. 50 bis ca. 95 % der Teilchen aus Natrium oder Kaliumpolyakrylat und ca. 5 bis ca. 50% der absorbierenden Teilchen auf der Basis von Weizenstärke enthält.

10. Ein absorbierender Einwegartikel übereinstimmend mit irgendeinem der vorhergenden Ansprüche **dadurch gekennzeichnet, dass** die äusseren Sperrschichtenn von einem Film nichtgewebter Struktur mit einem Gewicht zwischen ca. 10 und ca. 15 g/m² gebildet werden, und zwar aus einem Bikomponentenkern und Ummantelungsfasern, wobei der Kern ca. 80 bis ca. 100% Polymilchsäure enthält und die Ummantelung ca. 80 bis ca. 100% Polypropylen.

11. Ein absorbierender Einwegartikel übereinstimmend mit irgendeinem der Ansprüche 1 bis 10 **dadurch gekennzeichnet, dass** die äusseren Sperrschichtenn aus einer nichtgewebten Struktur mit einem Gewicht von etwa 10 bis etwa 15 g/m² geformt werden, gebildet aus einem Bikomponentenkern und Ummantelungsfasern, wobei der Kern etwa 80 bis 100% Poly-Hydroxialkanoate enthält und die Ummantelung etwa 80 bis 100% Polypropylen.

12. Ein absorbierender Einwegartikel übereinstimmend mit irgendeinem der Ansprüche 1 bis 10 **dadurch gekennzeichnet, dass** die äusseren Sperrschichtenn aus einer nichtgewebten dünnen Schicht mit einem Gewicht von ca. 10 bis ca. 15 g/m² geformt werden, die mindestens 15% Baumwollfasern enthält.

13. Ein absorbierender Einwegartikel übereinstimmend mit irgendeinem der vorhergenden Ansprüche **dadurch gekennzeichnet, dass** die elastischen Mittel des Artikels für Beine und Unterkörper aus Latex oder Naturkautschuk sind.

14. Ein absorbierender Einwegartikel übereinstimmend mit irgendeinem der Ansprüche 1 bis 12 **dadurch gekennzeichnet, dass** die elastischen Mittel des Artikels für Beine und den Unterkörper aus Polyolefinen zusammen mit einem degradierungsfördernden Agens hergestellt werden.

15. Ein absorbierender Einwegartikel übereinstimmend mit irgendeinem der vorhergenden Ansprüche **dadurch gekennzeichnet, dass** das Befestigungssystem des Artikels aus einem Paar Befestigungstreifen besteht, die an der Lendenzone angebracht werden, und einem Band, das ausserhalb des Bauchbereichs desselben angebracht wird, wobei die Befestigungsstreifen und/oder -bänder aus Polyolefinen zusammen mit einem degradierungsfördernden Agens geformt werden, der verseifte Fettsäuren und Kalzium enthält.

16. Ein absorbierender Einwegartikel übereinstimmend mit irgendeinem der Ansprüche 1 bis 14 **dadurch gekennzeichnet, dass** das Befestigungssystem mit einem Paar Befestigungsstreifen gebildet wird, die am Gegenstand entsprechend der Lendenzone angebracht werden, und aus einem Band, das auf der dem Bauch entsprechenden Aussenseite des Gegenstandes angebracht wird, wobei die Befestigungsstreifen und/oder das Band Polymilchsäure oder Poly-Hydroxy-Alkanoate enthalten.

17. Ein absorbierender Einwegartikel übereinstimmend mit irgendeinem der vorhergenden Ansprüche **dadurch gekennzeichnet, dass** er Dehnbereiche umfasst, die aus einem elastischem Polymer bestehen, das zwischen zwei dünnen Schichten eingebettet wird; das Elastomermaterial ist ein Polyolefin einschliesslich eines degradierungsfördernden Agens, der verseifte Fettsäuren und Kalzium enthält, und die dünnen Schichten sind nichtgewebte Schichten.

18. Ein absorbierender Einwegartikel übereinstimmend mit irgendeinem der Ansprüche 1 bis 16 **dadurch gekennzeichnet, dass** er Dehnbereiche aufweist, die aus elastischem Polymer bestehen, das zwischen zwei dünnen Schichten eingebettet wird; das Elastomermaterial ist ein Polyolefin einschliesslich eines degradierungsfördernden Agens, der verseifte Fettsäuren und Kalzium enthält, und die dünnen Schichten bestehen aus nichtgewebten Strukturen von etwa 10-15 g/m², die durch einen Bikomponentenkern und Ummantelunggsfasern gebildet werden, wobei der Kern ca. 80 bis ca. 100% Polymilchsäure enthält und die Ummantelung ca. 80 bis 100 % Polypropylen.

19. Ein absorbierender Einwegartikel übereinstimmend mit irgendeinem der Ansprüche 1 bis 16, **dadurch gekennzeichnet, dass** er Dehnbereiche aufweist, die aus einem elastischen Polymer bestehen, das zwischen zwei dünnen Schichten eingebettet ist; das Elastomermaterial ist ein Polyolefin einschliesslich eines degradierungsfördernden Agens, der verseifte Fettsäure und Kalzium enthält, und die dünnen Schichten sind nichtgewebte Strukturen von ca. 10.15 g/m², die durch einen Bikomponentenkern und Ummantelungsfasern gebildet werden, wobei der Kern etwa 80 bis etwa 100 % Poly-Hydroxy-Alkanoat enthält und die Ummantelung ca. 80 bis 100 % Polypropylen.

20. Ein absorbierender Einwegartikel übereinstimmend mit irgendeinem der Ansprüche 1 bis 17 **dadurch gekennzeichnet, dass** er Dehnbereiche aufweist, die aus einem elastischen Polymer bestehen, das zwischen zwei dünnen Schichten eingebettet ist; das Elastomermaterial ist ein Polyolefin einschliesslich eines degradierungsfördernden Agens mit verseiften Fettsäuren und Kalzium, und die dünnen Schichten sind nichtgewebte Strukturen von ca. 10-15 g/m² und mit mindestens 20% Baumwollfasern.

21. Ein absorbierender Einwegartikel übereinstimmend mit irgendeinem der vorhergenden Ansprüche **dadurch gekennzeichnet, dass** er eine Verbindung für die Hautpflege enthält, wobei es sich um eine Formel mit Pflanzenölen handelt, hauptsächlich Avocadoöl und süsses Mandelöl.

22. Ein absorbierender Einwegartikel übereinstimmend mit irgendeinem der vorhergenden Ansprüche **dadurch gekennzeichnet, dass** er eine Verbindung für die Hautplege enthält, wobei es sich um eine Formel handelt, die eine Mischung von aetherischen Ölen umfasst.

23. Ein absorbierender Einwegartikel übereinstimmend mit irgendeinem der vorhergenden Ansprüche **dadurch gekennzeichnet, dass** er Chlorophyll oder Chlorophyllderivate als Mittel für die Geruchskontrolle enthält.

## Revendications

1. Un article absorbant jetable, comprenant une feuille supérieure perméable, une feuille inférieure imperméable, un noyau absorbant placé entre les feuilles inférieure et supérieure contenant des fibres de matière absorbante et des particules de matière super-absorbante, des fronces barrières extérieures, des éléments élastiques aux jambes et à la taille, un système de fixation et des éléments de liaison; **caractérisé en ce qu'**au moins 50 % desdits éléments contiennent des composés biodégradables, ces composés biodégradables étant faits de composés naturels, de sorte qu'après une certaine période ceux-ci se dégradent et se transforment en composés moins complexes jusqu'à atteindre un état des plus basiques, et où ces composés biodégradables ont été sélectionnés dans :
pour la feuille supérieure de l'article: un tissu non tissé contenant du coton, un tissu non tissé contenant de l'acide polylactique (PLA) ou des polyhydroxyalcanoates (PHA) ou un tissu non tissé contenant un agent pro-dégradant ;
pour la feuille inférieure de l'article: un film étanche à l'eau contenant de l'acide polylactique (PLA) ou des polyhydroxyalcanoates (PHA) ou du polyéthylène contenant un agent pro-dégradant ; ou une couche laminée de film étanche à l'eau et un tissu non tissé où le film étanche à l'eau inclut du PLA, des PHA ou du polyéthylène contenant un agent pro-dégradant, et où le tissu non tissé inclut du PLA, des PHA, du coton ou un agent pro-dégradant;
en tant que matière du noyau absorbant: des fibres de cellulose ;
en tant que matière super-absorbante : des particules contenant au moins 20 % de particules biodégradables;
en tant que fronces barrières: un tissu non tissé contenant du PLA, des PHA ou un agent pro-dégradant ; des éléments élastiques : du latex, du caoutchouc naturel ou des matières élastiques contenant des polyoléfine plus un agent pro-dégradant;
en tant que système de fixation: deux bandes et une bande frontale faites en PHA ou PLA ou polyéthylène ou polypropylène contenant un agent pro-dégradant;
en tant qu'élément de liaison: des adhésifs contenant des PHA ou du PLA.
et où la feuille supérieure de l'article est un tissu non tissé avec un poids de 10 à 15 g/m² formé à partir des fibres à deux composants du noyau et de la gaine, le noyau contenant 80 à 100 % d'acide polylactique, et la gaine contenant 80 à 100 % de polypropylène.

2. Un article absorbant jetable comme décrit dans la revendication précédente, **caractérisé en ce que** la feuille inférieure imperméable est un film à faible densité en polyéthylène avec un poids d'environ 10-25 g/m² et contenant un agent pro-dégradant qui contient des acides gras saponifiés et du calcium.

3. Un article absorbant jetable comme décrit dans les revendications 1-2, caractérisé en que la feuille inférieure imperméable est un film fait à partir d'acide polylactique.

4. Un article absorbant jetable comme décrit dans les revendications 1-2, **caractérisé en ce que** la feuille inférieure imperméable est un film formé de polyhydroxyalcanoates.

5. Un article absorbant jetable comme décrit dans les revendications 1-2, **caractérisé en ce que** la feuille inférieure imperméable est laminée avec un tissu non tissé d'environ 10 et 15 g/m² et contenant un noyau à deux composants et des fibres de gaine, avec le noyau contenant entre 80 et 100 % environ d'acide polylactique, et la gaine contenant entre 80 et 100 % environ de polypropylène.

6. Un article absorbant jetable comme décrit dans les revendications 1-2, **caractérisé en ce que** la feuille inférieure imperméable est laminée avec un tissu non tissé d'environ 10 et 15 g/m² et contenant un noyau à deux composants et des fibres de gaine, le noyau contenant entre 80 et 100 % environ de polyhydroxyalcanoates et la gaine contenant entre 80 et 100 % environ de polypropylène.

7. Un article absorbant jetable comme décrit dans les revendications 1-2, **caractérisé en ce que** la feuille inférieure imperméable est laminée avec un tissu non tissé d'environ 10 et 15 g/m² et contenant au moins environ 20 % de fibres de coton.

8. Un article absorbant jetable comme décrit dans les revendications précédentes, **caractérisé en ce que** le noyau super-absorbant est formé de fibres cellulose et de deux types de particules, mélangées de façon homogène :
a) Particules de polyacrylate de sodium ou potassium
b) Particules absorbantes issues de la fécule de maïs

9. Un article absorbant jetable comme décrit dans la revendication 8, **caractérisé en ce que** le mélange contient entre 50 % et 95 % environ de particules de polyacrylate de sodium ou potassium et entre 5 % et 50 % environ de particules absorbantes issues de la fécule de maïs.

10. Un article absorbant jetable comme décrit dans les revendications précédentes, **caractérisé en ce que** les fronces barrières extérieures sont formées à partir d'un film de tissu non tissé avec un poids oscillant entre 10 et 15 mg/m², formé d'un noyau à deux composants et de fibres de gaine, le noyau contenant entre 80 et 100 % environ d'acide polylactique, et la gaine contenant entre 80 et 100 % environ de polypropylène.

11. Un article absorbant jetable comme décrit dans les revendications 1 à 10, **caractérisé en ce que** les fronces barrières extérieures sont formées à partir d'un tissu non tissé avec un poids oscillant entre 10 et 15 mg/m², formé d'un noyau à deux composants et de fibres de gaine, le noyau contenant entre 80 et 100 % environ de polyhydroxyalcanoates, et la gaine contenant entre 80 et 100 % environ de polypropylène.

12. Un article absorbant jetable comme décrit dans les revendications 1 à 10, **caractérisé en ce que** les fronces barrières extérieures sont formées à partir d'un film non-tissé avec un poids oscillant entre 10 et 15 mg/m² et contenant au moins 15 % de fibres de coton.

13. Un article absorbant jetable comme décrit dans les revendications précédentes, **caractérisé en ce que** les éléments élastiques de la jambe et de la taille de l'article sont en latex ou caoutchouc naturel.

14. Un article absorbant jetable comme décrit dans les revendications 1 à 12, **caractérisé en ce que** les éléments élastiques de la jambe et de la taille de l'article sont des polyoléfines et intègrent un agent pro-dégradant.

15. Un article absorbant jetable comme décrit dans les revendications précédentes, **caractérisé en ce que** le système de fixation de l'article consiste en deux rubans de fixation placés à l'arrière de la taille, et en une bande placée à l'extérieur du devant de la taille, et ces rubans de fixation et / ou la bande sont des polyoléfines et intègrent un agent pro-dégradant contenant des acides gras saponifiés et du calcium.

16. Un article absorbant jetable comme décrit dans les revendications 1 à 14, **caractérisé en ce que** le système de fixation consiste en deux rubans de fixation placés à l'arrière de la taille de l'élément, et en une bande placée à l'extérieur du devant de la taille, ces rubans de fixation et / ou la bande contenant de l'acide polylactique ou des polyhydroxyalcanoates.

17. Un article absorbant jetable comme décrit dans les revendications précédentes, **caractérisé en ce qu'**il contient des zones de stretch qui consistent en un matériau élastomère placé entre deux films : le matériau élastomère est une polyoléfine intégrant un agent pro-dégradant contenant des acides gras saponifiés et du calcium, et les films sont en tissu non tissé.

18. Un article absorbant jetable comme décrit dans les revendications 1 à 16, **caractérisé en ce qu'**il contient des zones de stretch qui consistent en un matériau élastomère placé entre deux films : le matériau élastomère est une polyoléfine intégrant un agent pro-dégradant contenant des acides gras saponifiés et du calcium, et les films en tissu non tissé d'environ 10-15 mg/m² sont formés d'un noyau à deux composants et de fibres de gaine, le noyau contenant entre 80 et 100 % environ d'acide polylactique, et la gaine contenant entre 80 et 100 % de polypropylène.

19. Un article absorbant jetable comme décrit dans les revendications 1 à 16, **caractérisé en ce qu'**il contient des zones de stretch qui consistent en un matériau élastomère placé entre deux films ; le matériau élastomère est une polyoléfine intégrant un agent pro-dégradant contenant des acides gras saponifiés et du calcium, et les films en tissu non tissé d'environ 10-15 mg/m² sont formés d'un noyau à deux composants et de fibres de gaine, le noyau contenant entre 80 et 100 % environ de polyhydroxyalcanoates, et la gaine contenant entre 80 et 100 % de polypropylène.

20. Un article absorbant jetable comme décrit dans les revendications 1 à 17, **caractérisé en ce qu'**il contient des zones de stretch qui consistent en un matériau élastomère placé entre deux films ; le matériau élastomère est une polyoléfine intégrant un agent pro-dégradant contenant des acides gras saponifiés et du calcium, et les films en tissu non tissé d'environ 10-15 mg/m² contiennent au moins environ 20 % de fibres de coton.

21. Un article absorbant jetable comme décrit dans les revendications précédentes, **caractérisé en ce qu'**il contient une composition apte au soin de la peau, qui est une formule contenant des huiles végétales, et principalement de l'huile d'avocat et de l'huile d'amande douce.

22. Un article absorbant jetable comme décrit dans les revendications précédentes, **caractérisé en ce qu'**il contient une composition apte au soin de la peau, qui est une formule contenant un mélange d'huiles essentielles.

23. Un article absorbant jetable comme décrit dans les revendications précédentes, **caractérisé en ce qu'**il contient de la chlorophylle ou des dérivés de chlorophylle en tant que moyen de contrôler les odeurs.
